(19) Europäisches Patentamt
European Patent Office
Office européen des brevets

(11) Numéro de publication: **0 061 380**
**A1**

# (12) DEMANDE DE BREVET EUROPEEN

(21) Numéro de dépôt: 82400398.2

(22) Date de dépôt: 08.03.82

(51) Int. Cl.³: C 07 D 487/04
A 61 K 31/505
//(C07D487/04, 239/00, 235/00)

(30) Priorité: 25.03.81 FR 8106006

(43) Date de publication de la demande: 29.09.82 Bulletin 82/39

(84) Etats contractants désignés: AT BE CH DE FR GB IT LI LU NL SE

(71) Demandeur: SYNTHELABO
58, rue de la Glacière
F-75621 Paris Cedex 13(FR)

(72) Inventeur: Binet, Jean
12, Hameau de la Gondole
F-91650 Breuillet(FR)

(72) Inventeur: Manoury, Philippe
9, rue de Malabry
F-92350 Le Plessis Robinson(FR)

(74) Mandataire: Thouret-Lemaitre, Elisabeth et al,
Service Brevets - SYNTHELABO 58, rue de la Glacière
F-75621 Paris Cedex 13(FR)

## (54) Dérivés d'imidazo (1,2-a) pyrimidines, leur préparation et leur application en thérapeutique.

(57) Imidazo [1,2-a] pyrimidines répondant à la formule (I)

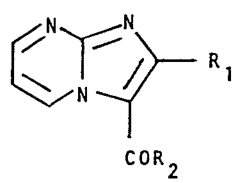

(1)

dans laquelle
$R_1$ et $R_2$ représentent indépendamment l'un de l'autre, soit un radical ($C_{1-4}$) alkyle, soit un radical ($C_{3-6}$) cycloalkyle, soit un radical phényle pouvant porter de 1 à 3 substituants choisis parmi les atomes d'halogène, les radicaux hydroxy, ($C_{1-4}$)-alkyles, ($C_{1-4}$)-alcoxy et $S(O))_m$-($C_{1-4}$)-alkyles où m est 0 ou 1, soit un radical pyridyle, soit un radical furyle, soit un radical tétrahydrofuryle,
l'un au moins des deux radicaux $R_1$ ou $R_2$ étant un radical phényle substitué ou non.

Application en thérapeutique.

EP 0 061 380 A1

DERIVES D'IMIDAZO [1,2-a] PYRIMIDINES, LEUR PREPARATION ET LEUR APPLICATION EN THERAPEUTIQUE.

La présente invention concerne des dérivés d'imidazo[1,2-a] pyrimidines, leur préparation et leur application en thérapeutique.

Les composés de l'invention répondent à la formule (I)

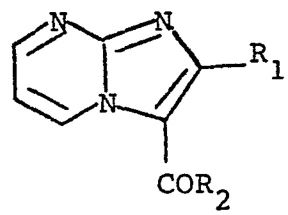

(I)

dans laquelle

$R_1$ et $R_2$ représentent indépendamment l'un de l'autre, soit un radical $(C_{1-4})$alkyle, soit un radical $(C_{3-6})$cycloalkyle, soit un radical phényle pouvant porter de 1 à 3 substituants choisis parmi les atomes d'halogène, les radicaux hydroxy, $(C_{1-4})$-alkyles, $(C_{1-4})$-alcoxy et $S(O)_m-(C_{1-4})$-alkyles où m est 0 ou 1, soit un radical pyridyle, soit un radical furyle, soit un radical tétrahydrofuryle,

l'un au moins des deux radicaux $R_1$ ou $R_2$ étant un radical phényle substitué ou non.

Les sels que forment les composes (I) avec les acides pharmaceutiquement acceptables font partie de l'invention.

Parmi les composés de l'invention, sont préférés ceux pour lesquels $R_2$ représente un radical $(C_{1-4})$alkyle et $R_1$ représente un radical phényle portant un substituant en position para, et plus particulièrement ceux pour lesquels $R_1$ représente un radical phényle portant un atome d'halogène ou un radical alkyle en position para et $R_2$ représente le radical éthyle.

Selon l'invention, on peut préparer les composés selon le schéma réactionnel suivant :

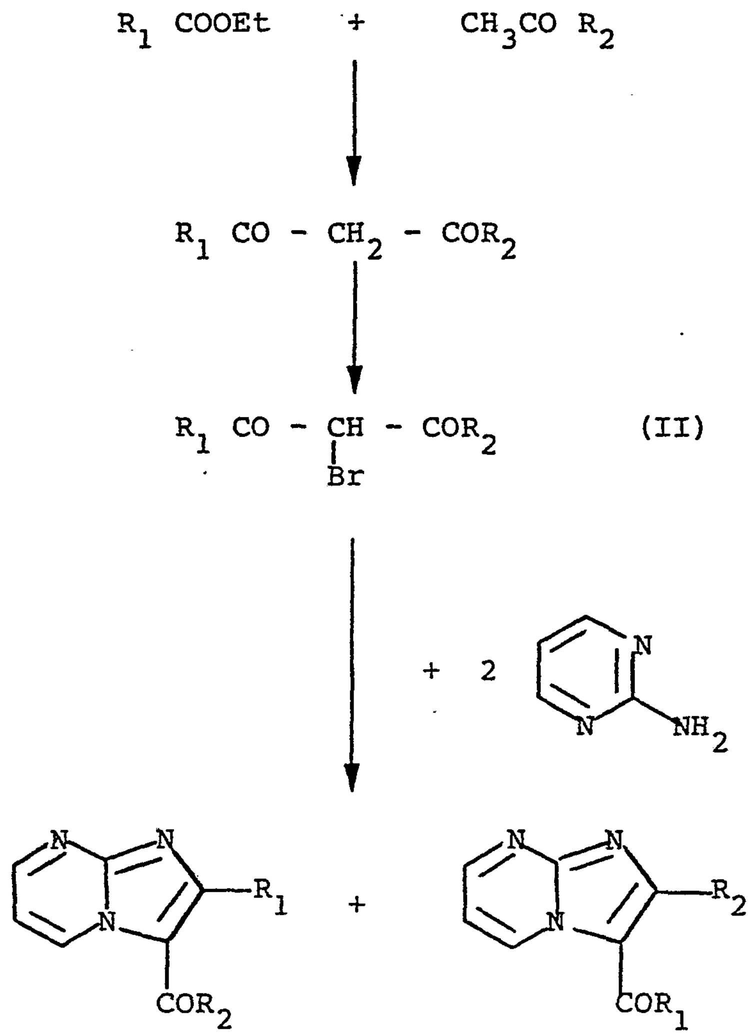

(II)

(I) (I bis)

La réaction entre le composé (II) et l'amino-2 pyrimidine conduit aux 2 composés de structures (I) et (Ibis).

La condensation est effectuée à une température de 20 à 100°C, dans un solvant tel que l'acétone, l'alcool, le DMF ou le HMPT.

La nature des substituants sur le noyau phényle influe sur le sens de la cyclisation. Si le substituant est un groupement hydroxyle, on obtient un seul composé. Dans les autres cas, on obtient, en proportions variables, un mélange d'isomères (I) et (I bis), qui peuvent être séparés par chromatographie ou par cristallisation fractionnée. L'un des composés étant beaucoup plus soluble dans l'éther que l'autre, le lavage du produit brut avec ce solvant permet une séparation

grossière en dissolvant l'un des isomères, l'autre restant à peu près insoluble. La purification se poursuit par cristallisation de la base ou du sel des deux produits séparés.

Les exemples suivants illustrent l'invention. Les analyses et les spectres IR et RMN confirment la structure des composés.
HMPT = hexaméthylphosphorotriamide.

EXEMPLE 1 [(Chloro-4 phényl)-2 imidazo[1,2-a]pyrimidinyl-3]-1 propanone-1 et son méthanesulfonate et (Chloro-4 phényl)(éthyl-2 imidazo[1,2-a]pyrimidinyl-3) méthanone et son méthanesulfonate.

A une suspension de 10,5 g (0,11 mole) d'amino-2 pyrimidine dans 50 $cm^3$ de HMPT, on ajoute rapidement 14,4 g (0,05 mole) de bromo-2 (chloro-4 phényl)-1 pentanedione-1,3.

1) On agite jusqu'à dissolution puis laisse au repos à la température ambiante pendant une nuit. On verse le mélange réactionnel sur un mélange eau + éther et agite jusqu'à cristallisation. On essore le précipité, le lave à l'eau et à l'éther et le sèche.
Aprés recristallisation de ce précipité dans de l'acétone anhydre, on obtient la [(chloro-4 phényl)-2 imidazo[1,2-a]pyrimidinyl-3]-1 propanone-1 sous forme de base qui fond à 198°C.

On prépare le méthanesulfonate par addition d'acide méthanesulfonique à la base dans de l'alcool bouillant.

F = 208°C

2) On rassemble les filtrats organiques obtenus après séparation des premiers cristaux puis les sèche sur $MgSO_4$. On concentre. On acidifie avec 10 $cm^3$ d'une solution 2N d'acide méthane-sulfonique dans le méthanol. On essore les cristaux formés. On les fait recristalliser dans de l'alcool absolu.

On obtient le méthanesulfonate de la (chloro-4 phényl)(éthyl-2 imidazo[1,2-a]pyrimidinyl-3) méthanone.

F = 224°C

On libère la base par alcalinisation à l'aide de $NaHCO_3$ d'une solution méthanolique du méthanesulfonate.

F = 158°C

EXEMPLE 2 [(Triméthoxy-3,4,5 phényl)-2 imidazo[1,2-a]pyrimidinyl-3]-1 propanone-1 et (Ethyl-2 imidazo[1,2-a]pyrimidinyl-3)(triméthoxy-3,4,5 phényl) méthanone.

On ajoute, par portions, 69 g (0,2 mole) de bromo-2 (triméthoxy-3,4,5 phényl)-1 pentanedione-1,3 à une suspension de 38 g (0,4 mole) d'amino-2 pyrimidine dans 150 $cm^3$ de HMPT. On agite le mélange jusqu'à obtention d'une solution puis on laisse au repos 2 jours.
On le verse sur un mélange d'eau et d'éther. On filtre les cristaux formés, les lave avec de l'eau puis avec de l'éther. On obtient après recristallisation dans de l'acétone la [(triméthoxy-3,4,5 phényl)-2 imidazo[1,2-a]pyrimidinyl-3]-1 propanone-1 qui fond à 195°C.
On évapore à sec le filtrat éthéré, dissout le résidu d'évaporation dans de l'acétone, acidifie la solution ainsi obtenue avec de l'éther chlorhydrique. On filtre les cristaux formés et libère la base avec du carbonate de sodium dans du méthanol. On obtient ainsi, après recristallisation, l' (éthyl-2 imidazo[1,2-a]pyrimidinyl-3)(triméthoxy-3,4,5 phényl) méthanone qui fond à 130°C.

EXEMPLE 3 (Chloro-4 phényl) [( pyridinyl-2 )-2 imidazo[1,2-a] pyrimidinyl-3] méthanone et [(Chloro-4 phényl)-2 imidazo[1,2-a]pyrimidinyl-3] (pyridinyl-2) méthanone.

On agite pendant 24h un mélange de 28,5 g (0,3 mole) d'amino-2 pyrimidine et de 52 g (0,15 mole) de bromo-2 (chloro-4 phényl)-3 (pyridinyl-2)-1 propanedione-1,3 dans 150 $cm^3$ de HMPT. On verse la solution sur un mélange d'eau et d'éther. On filtre le précipité obtenu, le lave avec de l'eau et de l'éther. On solubilise le produit dans de l'éthanol, acidifie avec une solution d'éthanol chlorhydrique et filtre les cristaux ainsi obtenus. On libère la base dans du méthanol par addition d'une solution aqueuse de bicarbonate de sodium normal. On obtient, après recristallisation dans de l'éthanol, la (chloro-4 phényl) [(pyridinyl-2)-2 imidazo[1,2-a]pyrimidinyl-3] méthanone qui fond à 201°C.

Au filtrat éthanolique acide obtenu précédemment, on ajoute un volume égal d'éther puis filtre les cristaux obtenus après deux jours de repos. On libère la base dans du méthanol par addition d'une solution aqueuse de bicarbonate de sodium normal. On obtient ainsi la [(chloro-4 phényl)-2 imidazo[1,2-a] pyrimidinyl-3](pyridinyl-2) méthanone qui fond, après recristallisation dans du métanol, à 214°C.

Dans le tableau suivant sont représentés les composés de l'invention préparés à titre d'exemples.

T A B L E A U

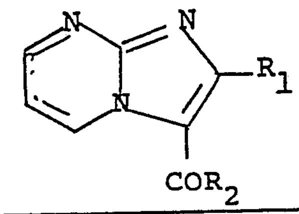

| Composé | $R_1$ | $R_2$ | F (°C) base ou sel |
|---|---|---|---|
| 1 | Et | 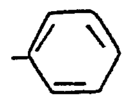 | 210-(HCl) |
| 2 | Et | 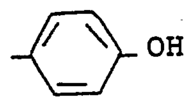 | 236 (HCl) |
| 3 (ex 1) | 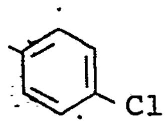 | Et | 208 ($Me-SO_3H$ |
| 4 (ex 1) | Et | 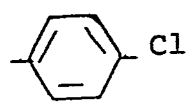 | 224 ($Me-SO_3H$) |
| 5 | 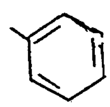 | Isopr. | 175° ($Me-SO_3H$) |
| 6 | Isopr. | 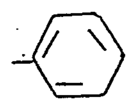 | 179° ($Me-SO_3H$) |
| 7 | 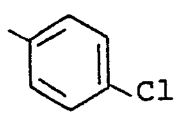 | 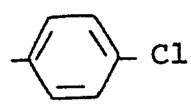 | 255° ($Me-SO_3H$) |
| 8 | Et | 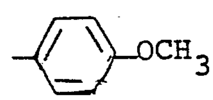 | 220° ($Me-SO_3H$) |
| 9 | Et | 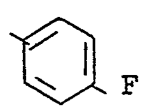 | 250° ($Me-SO_3H$) |
| 10 | 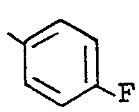 | Et | 221° ($Me-SO_3H$) |

T A B L E A U ( suite I) 

| Composé | $R_1$ | $R_2$ | F (°C) base ou sel |
|---|---|---|---|
| 11 | Me | 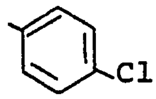 | 250° ($Me-SO_3H$) |
| 12 | 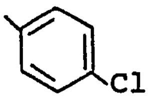 | Me | 184° ($Me-SO_3H$) |
| 13 | 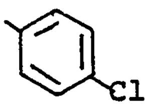 | Isopr. | 185° ($Me-SO_3H$) |
| 14 | 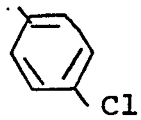 | 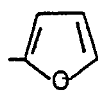 | 167° (base) |
| 15 | 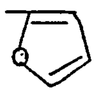 | 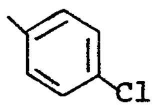 | 161° (base) |
| 16 | 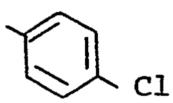 | 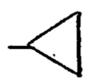 | 189° (base) |
| 17 | 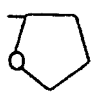 | 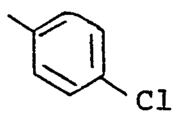 | 172° ($Me-SO_3H$) |
| 18 | 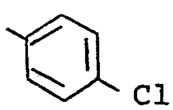 | n-But | 170° (base) |
| 19(ex 2) | Et | 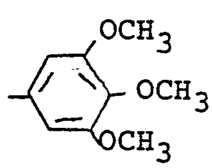 | 130° (base |
| 20(ex 2) | 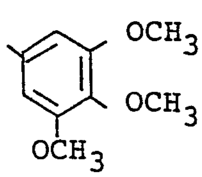 | Et | 195° (base) |
| 21(ex 3) | 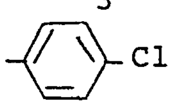 | 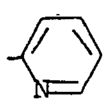 | 214° (base) |
| 22(ex 3) | 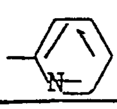 | 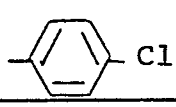 | 201° (base) |

TABLEAU (suite et fin)

| Composé | $R_1$ | $R_2$ | F (°C) base ou sel |
|---|---|---|---|
| 23 | 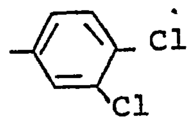 | Et | 207° (Me-$SO_3$H) |
| 24 | Et | 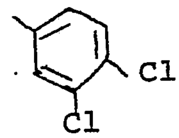 | 230° (Me-$SO_3$H) |
| 25 | 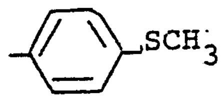 | Et | 220° (Me-$SO_3$H) |
| 26 | Et | 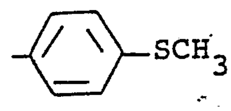 | 214° (Me-$SO_3$H) |
| 27 | Et | 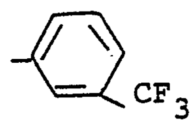 | 124° (base) |
| 28 | 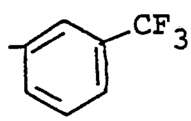 | Et | 151° (base) |
| 29 | 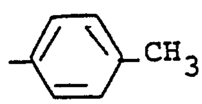 | Et | 141° (base) |
| 30 | Et | 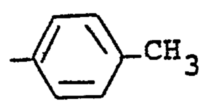 | 234° (Me-$SO_3$H) |
| 31 | 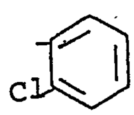 | Et | 171° (base) |
| 32 | Et | 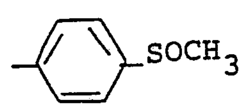 | 155° (base) |

Les composés de l'invention ont été soumis à des essais pharmacologiques qui ont montré leurs propriétés thérapeutiques dans le domaine du système nerveux central.

La toxicité des composés a été déterminée chez la souris après absorption orale. La DL 50 varie de 500 mg à 2000 mg/kg p.o.

L'action inductrice du sommeil et l'activité hypnogène ont été déterminées par observation de l'action des composés :

1) sur les états de veille-sommeil chez le rat implanté libre selon la méthode décrite par DEPOORTERE H., ROUSSEAU A. et JALFRE M. dans Rev. E.E.G. Neurophysiol. 7, 153-157 (1977).

Les composés de l'invention administrés à des doses de 3 à 10 mg/kg p.o. réduisent la durée totale de l'éveil sans modifier la durée du sommeil paradoxal ; la durée totale du sommeil paradoxal est seulement réduite après la dose de 30 mg/kg p.o.

2) sur les états de veille-sommeil chez le chat implanté libre selon la méthode décrite par DA COSTA L.M., DEPOORTERE H. et NAQUET R. dans Rev. E.E.G. Neurophysiol. 7, 158-164 (1977).

Les composés de l'invention, administrés à des doses de 3 à 10 mg/kg i.p. ou de 3 à 10 mg/kg p.o., augmentent la durée du sommeil à ondes lentes (S.O.L. et S.P.O.L.) sans réduire la durée du sommeil paradoxal ; la durée totale du sommeil paradoxal est seulement réduite après des doses de 20 mg/kg i.p. ou 30 mg/kg p.o.

Les résultats pharmacologiques montrent que les composés de l'invention peuvent être utilisés en thérapeutique comme inducteurs du sommeil et hypnotiques.

Ils peuvent être présentés sous la forme de compositions thérapeutiques destinées à l'administration par voie orale ou parentérale, en association avec tout excipient approprié.

La posologie quotidienne peut aller de 10 à 300 mg.

## Revendications

1. Imidazo[1,2-a]pyrimidines répondant à la formule (I)

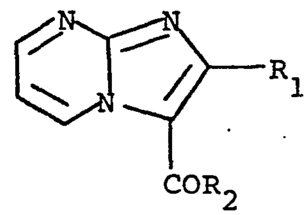

(I)

dans laquelle
$R_1$ et $R_2$ représentent indépendamment l'un de l'autre, soit un radical $(C_{1-4})$ alkyle, soit un radical $(C_{3-6})$cyclo-alkyle, soit un radical phényle pouvant porter de 1 à 3 substituants choisis parmi les atomes d'halogène, les radicaux hydroxy, $(C_{1-4})$-alkyles, $(C_{1-4})$-alcoxy et $S(O)_m$-$(C_{1-4})$-alkyles où m est O ou 1, soit un radical pyridyle, soit un radical furyle, soit un radical tétrahydrofuryle,
l'un au moins des deux radicaux $R_1$ ou $R_2$ étant un radical phényle substitué ou non,
ainsi que leurs sels d'addition aux acides pharmaceutiquement acceptables.

2. Dérivés selon la revendication 1, caractérisés par le fait que $R_2$ représente un radical $(C_{1-4})$alkyle et $R_1$ représente un radical phényle portant un substituant en position para.

3. Dérivés selon la revendication 2, caractérisés par le fait que $R_2$ représente le radical éthyle et $R_1$ représente un radical phényle portant un atome d'halogène ou un radical alkyle en position para.

4. La [(chloro-4 phényl)-2 imidazo[1,2-a]pyrimidinyl-3]-1 propanone-1 et ses sels.

5. Procédé de préparation des composés selon la revendication 1, procédé caractérisé en ce que l'on fait réagir une dicétone de formule (II)

$$R_1 CO - \underset{\displaystyle Br}{\underset{|}{CH}} - COR_2 \qquad (II)$$

avec l'amino-2 pyrimidine, $R_1$ et $R_2$ ayant les significations données dans la revendication 1.

6. Composition thérapeutique caractérisée en ce qu'elle contient un composé selon l'une quelconque des revendications 1 à 4 en association avec tout excipient approprié.

7. Médicament caractérisé en ce qu'il contient un composé selon l'une quelconque des revendications 1 à 4.

Office européen des brevets

**0061380**

Numéro de la demande

# RAPPORT DE RECHERCHE EUROPEENNE

EP 82 40 0398

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | CHEMICAL ABSTRACTS, vol. 86, no. 25, 25 juin 1977 réf.: 189839v, page 604 COLUMBUS, OHIO (US) & ARCH. PHARM. (WEINHEIM, GER.) 1976, 309(12), 959-65 H. BOEHME et al.: "β-substituted enamines, part. 8. Fused ring systems from 2-aminoheterocycles and derivatives of α-chloroaceto-acetic acid"<br>------ | 1,6 | C 07 D 487/04<br>A 61 K 31/505//<br>(C 07 D 487/04<br>239/00<br>235/00) |

DOMAINES TECHNIQUES RECHERCHES (Int. Cl. 3)

C 07 D 487/00
A 61 K 31/00

Le présent rapport de recherche a été établi pour toutes les revendications

| Lieu de la recherche | Date d'achèvement de la recherche | Examinateur |
|---|---|---|
| La Haye | 10-06-1982 | ALFARO |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503.03.82